(19) Europäisches Patentamt
European Patent Office
Office européen des brevets

(11) Veröffentlichungsnummer: **0 133 913**
**B1**

# (12) EUROPÄISCHE PATENTSCHRIFT

(45) Veröffentlichungstag der Patentschrift: **07.10.87**

(51) Int. Cl.⁴: **C 07 C 21/09**, C 07 C 17/10, C 07 C 21/04, C 07 C 17/02

(21) Anmeldenummer: **84107639.1**

(22) Anmeldetag: **02.07.84**

## (54) Verfahren zur Herstellung von 3,3-Dichlor-2-methyl-propen.

(30) Priorität: **25.08.83 DE 3330610**
**25.04.84 DE 3415337**

(43) Veröffentlichungstag der Anmeldung: **13.03.85 Patentblatt 85/11**

(45) Bekanntmachung des Hinweises auf die Patenterteilung: **07.10.87 Patentblatt 87/41**

(84) Benannte Vertragsstaaten: **AT BE CH DE FR GB IT LI NL**

(56) Entgegenhaltungen:
**CHEMISCHES ZENTRALBLATT, Band 110, Nr. 25, 1939, Seiten 4221-4223, Berlin; D.W. TISCHTSCHENKO "Untersuchungen auf dem Gebiet der Chlorderivate der Fettreihe. XIV. Die additive Fähigkeit der Doppelbindung am quaternären Kohlenstoff", & CHEM. J. SER. A. J. ALLG. CHEM., Band 8, Seiten 1232-1246**
**JOURNAL OF THE AMERICAN CHEMICAL SOCIETY, Band 72, Nr. 8, 15. August 1950, Seiten 3577-3579, Columbus, Ohio, US; A.L. HENNE et al.: "The preparation of hexafluoroacetone"**
**JOURNAL OF THE AMERICAN CHEMICAL SOCIETY, Band 68, Nr. 5, 11. Mai 1946, Seiten 785-789, Columbus, Ohio, US: A. MOORADIAN et al.: "4-Chloro-3-methyl-3-butenonitrile and related compounds"**

(73) Patentinhaber: **HÜLS AKTIENGESELLSCHAFT, - RSP Patente / PB 15 - Postfach 13 20, D-4370 Marl 1 (DE)**

(72) Erfinder: **Müller, Dieter Jürgen, Dr., Stargarder Strasse 30, D-4370 Marl (DE)**

**EP 0 133 913 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 3,3-Dichlor-2-methyl-propen durch Umsetzung von 1-Chlor-2-methylpropen mit Sulfurylchlorid.

3,3-Dichlor-2-methyl-propen ist aufgrund seiner funktionellen Gruppen, nämlich sowohl geminalen Chlorsubstituenten als auch einer Doppelbindung als Zwischenprodukt für organische Synthesen interessant.

Dieses Produkt ist bisher kaum zugänglich, da es bei der technischen Gasphasen-Chlorierung von Isobuten weder als Haupt- noch als Nebenprodukt anfällt.

Die Herstellung von 3,3-Dichlor-2-methylpropen gelingt mit geringer Ausbeute von etwa 6% durch Einleiten von Chlorgas im stöchiometrischen Überschuss in siedendes 1-Chlor-2-methyl-propen unter Lichtausschuss (A.L. Henne et al., J. Am. Chem. Soc. 72 (1950) 3577).

Dieses Verfahren eignet sich wegen der geringen Selektivität nicht für eine technisch befriedigende Herstellung.

Es ist auch bekannt, 3,3-Dichlor-2-methylpropen dadurch herzustellen, dass man 1-Chlor-2-methyl-propen mit Chlor in Gegenwart von $NaHCO_3$ im Molverhältnis 1:1:1,5 bei 0 °C umsetzt, wobei neben 68% 3,3-Dichlor-2-methylpropen 32% 1,1,2-Trichlor-2-methyl-propan entstehen (Chem. Abstr. 33 (1939) 4190).

Dieses Verfahren ist ebenfalls unbefriedigend, da sowohl der Einsatz als auch die Abtrennung eines Feststoffes und darüber hinaus eine aufwendige Kühlung notwendig ist. Auch bei diesem Verfahren ist die Ausbeute unbefriedigend.

Der Erfindung liegt die Aufgabe zugrunde, ein Verfahren bereitzustellen, das die Herstellung von 3,3-Dichlor-2-methyl-propen in einfacher Weise in höheren Ausbeuten ermöglicht.

Diese Aufgabe wird erfindungsgemäss dadurch gelöst, dass man 1-Chlor-2-methyl-propen in Flüssigphase in Gegenwart von Aminen, wie Stickstoff-Basen, oder Phosphinen als Katalysatoren mit Sulfurylchlorid umsetzt.

Der Ablauf der diesem Verfahren zugrunde liegenden Reaktion

$$Cl{-}CH{=}\overset{\displaystyle CH_3 \atop |}{C}{-}CH_3 + SO_2Cl_2 \xrightarrow{kat.} CH_2{=}\overset{\displaystyle CH_3 \atop |}{C}{-}CHCl_2 + SO_2 + HCl$$

ist völlig überraschend.

Normalerweise reagiert $SO_2Cl_2$ mit ungesättigten aliphatischen Verbindungen entweder durch Addition von Chlor an die Doppelbindung und durch Freisetzen von $SO_2$ oder durch Addition der ganzen Molekel unter Bildung von Chloralkansulfonsäurechloriden. Beispielsweise entsteht aus dem isomeren 3-Chlor-2-methyl-propen mit $SO_2Cl_2$ durch Chloraddition das zu erwartende 1,2,3-Trichlor-2-methyl-propan.

Bei dem erfindungsgemässen Verfahren findet dagegen überraschenderweise überwiegend Substitution bei gleichzeitiger Doppelbindungsisomerisierung statt, wenn die Reaktion in Gegenwart von organischen Stickstoff-Basen und/oder organischen Phosphinen wie Aminen, insbesondere von aliphatischen, aromatischen oder heterozyklischen Stickstoffverbindungen bzw. von aliphatischen oder aromatischen Phosphinen durchgeführt wird.

Die Umsetzung von 1-Chlor-2-methyl-propen in Gegenwart von organischen Stickstoff-Basen und/oder organischen Phosphinen mit Sulfurylchlorid verläuft eoxtherm mit grosser Geschwindigkeit.

Es ist daher für eine ruhige Reaktionsführung zweckmässig, die Umsetzung in der Weise durchzuführen, dass man 1-Chlor-2-methyl-propen nach Zugabe der organischen Stickstoff-Basen bzw. der organischen Phosphine und Aufheizen auf eine Starttemperatur von 30 °C bis 65 °C Sulfurylchlorid unter Kühlung der Reaktionsmischung in dem Masse zudosiert, wie es abreagiert. Die Temperatur der Reaktionsmischung kann dabei bis an den Siedepunkt heranreichen. Die Abfuhr der Reaktionswärme kann sowohl über eine Mantelkühlung als auch über eine Verdampfungs-/Rückflusskühlung erfolgen. Entsprechend der Reaktionsgleichung entstehen aus Sulfurylchlorid nur die gasförmigen Nebenprodukte $SO_2$ und HCl, die laufend aus der Reaktionsmischung ausgasen und abgeführt werden. Zur Umsetzung kann man das Sulfurylchlorid in stöchiometrischen Mengen, bezogen auf das stöchiometrische Verhältnis zum 1-Chlor-2-methyl-propen, einsetzen.

Zweckmässigerweise wählt man einen Unterschuss an Sulfurylchlorid, bezogen auf das stöchiometrische Verhältnis zum 1-Chlor-2-methylpropen, da nicht umgesetztes 1-Chlor-2-methylpropen leichter aus dem resultierenden Produkt abtrennbar ist als nicht umgesetztes Sulfurylchlorid. Nicht umgesetztes 1-Chlor-2-methyl-propen kann nach Abtrennung erneut eingesetzt werden, so dass letztlich ein vollständiger Umsatz erreicht wird. Allerdings ist es für die Reaktion selbst auch nicht nachteilig, wenn $SO_2Cl_2$ im stöchiometrischen Überschuss, bezogen auf das stöchiometrische Verhältnis zum 1-Chlor-2-methyl-propen, eingesetzt wird.

Die Reaktion kann sowohl diskontinuierlich beispielsweise in einem Rührkessel als auch kontinuierlich beispielsweise in einem Rohrreaktor oder einer Kaskade durchgeführt werden. Bevorzugt führt man die Umsetzung in der Flüssigphase durch.

Als organische Stickstoff-Basen sind alle N-haltigen organischen Verbindungen mit freiem Elektronenpaar geeignet, beispielsweise aliphatische Amine, wie Diisopropylamin, Triethylamin

oder aromatische Amine, wie Diphenylamin oder heterozyklische Amine, wie Pyridin, Picoline, Pyrrol, Pyrazol oder Chinolin. Ebenso sind Kombinationen mehrerer Stickstoff-Basen möglich.

Bevorzugt setzt man Pyridin, Picolin, Diisopropylamin, Triethylamin und/oder Chinolin ein.

In der Regel genügen etwa 1 bis 10 000 ppm dieser Verbindungen, um die Reaktion in der gewünschten Weisezu katalysieren. Bevorzugt setzt man 10 bis 1000 ppm ein.

Da vielfach Chlorkohlenwasserstoffe mit Aminen stabilisiert werden, kann gegebenenfalls auf diese Weise stabilisiertes 1-Chlor-2-methyl-propen bereits in der gewünschten Weise reagieren, ohne dass die zusätzliche Zugabe einer Stickstoff-Base erforderlich wird.

Die aliphatischen oder aromatischen Phosphine setzt man in der Regel ebenfalls in einem Konzentrationsbereich von 1 bis 10 000 ppm, bevorzugt von 10 bis 1000 ppm, ein. Besonders geeignet sind aliphatische Phosphine. Auch Kombinationen mehrerer Phosphine sowie Kombinationen von Aminen mit Phosphinen sind geeignet.

Die Umsetzung erfolgt im allgemeinen bei einer Temperatur von 30 bis 68 °C, vorzugsweise 45 bis 65 °C in einer Zeit von 5 bis 200 min, vorzugsweise 30 bis 120 min. Die Zudosierzeit von $SO_2Cl_2$ beträgt im allgemeinen 1 bis 30 min, vorzugsweise 3 bis 15 min.

Zur Isolierung des 3,3-Dichlor-2-methyl-propen wird das Reaktionsprodukt einer üblichen Fraktionierung unterworfen, wobei es sich im Falle einer Normaldruckfraktionierung um die Fraktion mit dem Siedebereich von etwa 108 bis 112 °C handelt. Vorzugsweise wird die Fraktionierung allerdings unter vermindertem Druck durchgeführt, da das 3,3-Dichlor-2-methyl-propen bei thermischer Belastung zur Umlagerung in 1,3-Dichlor-2-methyl-propen neigt.

Die Ausbeuten des erfindungsgemässen Verfahrens an 3,3-Dichlor-2-methyl-propen liegen im allgemeinen über 80%, bezogen auf umgesetztes 1-Chlor-2-methyl-propen. Die Ausbeuten variieren etwas mit der Temperaturführung, der Zudosiergeschwindigkeit von Sulfurylchlorid, der Wahl des Katalysators, der Reaktionszeit und der Einwirkung von Licht.

Lichteinwirkung kann die Reaktion in Richtung 1,1,2-Trichlor-2-methyl-propan verschieben. Bei geeigneter Parameterwahl, beispielsweise bei einer Temperatur von 45 bis 50 °C, einer Zudosierzeit von Sulfurylchlorid von 1 bis 5 min, einer Reaktionszeit von 60 bis 120 min, ohne Einwirkung von Licht, sind Ausbeuten von über 90% möglich.

Die folgenden Beispiele sollen das erfindungsgemässe Verfahren verdeutlichen.

Beispiel 1

In einer Rührapparatur mit Rückflusskühler und Tropftrichter werden 87 g frisch destilliertes 1-Chlor-2-methyl-propen vorgelegt, mit einer Stickstoff-Base entsprechend Tabelle 1 versetzt, auf 45 °C erwärmt und dann insgesamt 108 g Sulfurylchlorid unter Rühren in 3 bis 30 Minuten zudosiert, wobei die Temperatur durch Thermostatisierung des Rührkolbens im Bereich zwischen 45 und etwa 65 °C gehalten wird. Die bei der Reaktion gebildeten gasförmigen Nebenprodukte $SO_2$ und HCl werden über den Rückflusskühler abgezogen und in einer Vorlage mit auf pH 9,5 alkalisiertem Wasser adsorbiert. Der pH-Wert wird durch automatische Titration mittels Natronlauge definierter Konzentration konstant gehalten, so dass der Reaktionsfortschritt anhand des Natronlaugeverbrauches verfolgt werden kann. Die Reaktionszeit liegt je nach Reaktionsbesdindungen zwischen 30 und 180 min, wobei Sulfurylchloridumsätze zwischen 80 und 98% erreicht werden.

Die resultierende Rohproduktmenge liegt je nach Reaktionsbedingungen zwischen etwa 115 g und 130 g. Das Produkt ist nach Wasserwäsche und Trocknung über $K_2CO_3$ einer gaschromatografischen Analyse unterworfen worden. Die resultierenden Reinproduktzusammensetzungen sind Tabelle 1 zu entnehmen, wobei das überschüssige, nicht umgesetzte 1-Chlor-2-methyl-propen rechnerisch eliminiert wird. Der Gehalt an gewünschtem 3,3-Dichlor-2-methyl-propen liegt zwischen 80 und 92%.

Die Versuchsbeispiele sind nicht hinsichtlich der Reaktionszeit und der Ausbeute optimiert worden, da sie den prinzipiellen Reaktionsablauf unter den erfindungsgemässen Bedingungen aufzeigen sollen. Auf den über die Titration des gebildeten $SO_2$ und HCl verfolgten Umsatzgeschwindigkeiten an $SO_2Cl_2$ ergibt sich, dass insbesondere bei Pyridin, Picolin, Diisopropylamin und Triethylamin Reaktionszeiten von nur 25 min ausreichend sind, um über 95% Umsatz zu erreichen.

Tabelle 1
Beispiele zur Umsetzung von 1-Chlor-2-methyl-propen mit Sulfurylchlorid im Unterschuss in Gegenwart von Stickstoffbasen

| Stickstoffbase, Zugabe ppm | Reaktionstemperatur °C | Zudosierzeit von $SO_2Cl_2$ min | Reaktionszeit min | Produktmenge g | Zusammensetzung des Reinproduktes*) %: 3,3-Dichlor-2-methylpropen | 1,3-Dichlor-2-methylpropen | 1,1,2-Trichlor-2-methylpropan |
|---|---|---|---|---|---|---|---|
| Pyridin 1000 | 40 bis 50 | 15 | 60 | 115 | 80,5 | 1,0 | 16,5 |
| Pyridin 100 | 45 | 30 | 120 | 120 | 84,0 | 1,2 | 11,0 |
| Chinolin 100 | 45 | 15 | 120 | 121 | 85,0 | 1,3 | 9,5 |

Tabelle 1

| Stickstoffbase, Zugabe ppm | Reaktions-temperatur °C | Zu-dosier-zeit von $SO_2Cl_2$ min | Reak-tions-zeit min | Pro-dukt-menge g | Zusammensetzung des Reinproduktes*) % 3,3-Di-chlor-2-methyl-propen | 1,3-Di-chlor-2-methyl-propen | 1,1,2-Tri-chlor-2-methyl-propan |
|---|---|---|---|---|---|---|---|
| α-Picolin 100 | 45 | 10 | 90 | 119 | 84,5 | 1,3 | 10,0 |
| γ-Picolin 100 | 45 | 10 | 60 | 118 | 87,0 | 0,8 | 8,5 |
| Pyrrol 1000 | 45 | 3 | 120 | 121 | 91,5 | 0,5 | 5,5 |
| Pyrazol 1000 | 45 | 10 | 180 | 130 | 80,5 | 1,0 | 17,0 |
| Diohenylamin 1000 | 45 | 10 | 60 | 118 | 81,5 | 1,5 | 15,0 |
| Triethylamin 1000 | 45 | 10 | 60 | 119 | 80,0 | 1,1 | 15,5 |
| Diisopropylamin 100 | 45 bis 55 | 15 | 120 | 124 | 80,0 | 1,2 | 16,5 |

*) Rest nicht identifiziert

Beispiel 2

Es wird wie im Beispiel 1 verfahren, jedoch mit dem Unterschied, dass zunächst keine Stickstoff-Base zugegeben wird und das gesamte $SO_2Cl_2$ sofort zugegeben wird. Die Reaktion ist äusserst träge und springt auch innerhalb 60 min nicht in der aus Beispiel 1 bekannten Weise an. Nach Zugabe von a) 0,1% Pyridin bzw. b) 0,1% Pyrazol springt die Reaktion sofort in der bekannten Weise an, wobei nach 180 min Reaktionszeit folgende Produktspektren erhalten worden sind:

a) 77,5% 3,3-Dichlor-2-methyl-propen,
0,5% 1,3-Dichlor-2-methyl-propen,
18,0% 1,1,2-Trichlor-2-methyl-propan,

b) 78,5% 3,3-Dichlor-2-methyl-propen,
1,0% 1,3-Dichlor-2-methyl-propan,
18,5% 1,1,2-Trichlor-2-methyl-propan.

Beispiel 3

Es wird wie im Beispiel 1 verfahren, allerdings wird der Umsatz an $SO_2Cl_2$ durch Titration des ausgasenden $SO_2$ und HCl nach Adsorption in wässriger NaOH quantitativ verfolgt.

Die Zudosierzeit des Sulfurylchlorids beträgt 10 min. Als Stickstoffbase werden 1000 ppm Triethylamin gewählt.

Nach 10 min, also bei Beendigung der $SO_2Cl_2$-Zugabe, beträgt der $SO_2Cl_2$-Umsatz 86%, nach 20 min 95% und nach 30 min 97%.

Beispiel 4

In der im Beispiel 1 beschriebenen Rührapparatur wird frisch destilliertes 1-Chlor-2-methyl-propen vorgelegt, mit einem Phosphin entsprechend Tabelle 2 versetzt und entsprechend den weiteren Ausführungen in Beispiel 1 verfahren.

Die Ergebnisse zeigt Tabelle 2.

Tabelle 2

Beispiel zur Umsetzung von 1-Chlor-2-methyl-propen mit $SO_2Cl_2$ in Gegenwart von Phosphin

| Phosphin-zugabe ppm | Reaktions-temperatur °C | Zudo-sierzeit von $SO_2Cl_2$ min | Reaktions-zeit min | Roh-produkt-menge g | Zusammensetzung des Reinproduktes +) 3,3-Dichlor-2-methyl-propen | 1,3-Dichlor-2-methyl-propen | 1,1,2-Tri-chlor-2-methyl-propan |
|---|---|---|---|---|---|---|---|
| Tributyl-phosphin 1000 | 45 bis 50 | 60 | 120 | 117 | 80,5 | 1,0 | 16,0 |
| Triphenyl-phosphin 1000 | 45 bis 50 | 60 | 120 | 115 | 75,0 | 2,0 | 21,0 |

+) Rest nicht identifiziert

**Patentansprüche**

1. Verfahren zur Herstellung von 3,3-Dichlor-2-methyl-propen, dadurch gekennzeichnet, dass man 1-Chlor-2-methyl-propen mit Sulfurylchlorid in Gegenwart katalytisch wirkender Mengen an organischen Stickstoff-Basen und/oder organischen Phosphinen umsetzt.

2. Verfahren nach Anspruch 1, dadurch gekennzeichnet, dass die Reaktion in Flüssigphase durchgeführt wird.

3. Verfahren nach Anspruch 1 und 2, dadurch gekennzeichnet, dass die Reaktion bei 30 bis 68 °C unter Normaldruck durchgeführt wird.

4. Verfahren nach Anspruch 1 bis 3, dadurch gekennzeichnet, dass Sulfurylchlorid, bezogen auf 1-Chlor-2-methyl-propen, im Über- oder Unterschuss, bezogen auf das stöchiometrische Verhältnis zum 1-Chlor-2-methyl-propen, eingesetzt wird.

5. Verfahren nach Anspruch 1 bis 4, dadurch gekennzeichnet, dass die organische Stickstoff-Base oder das organische Phosphin im Konzentrationsbereich von 1 bis 10 000 ppm dem 1-Chlor-2-methyl-propen zugesetzt wird oder 1-Chlor-2-methyl-propen mit diesen Konzentrationen an organischen Stickstoff-Basen und/oder organischen Phosphinen eingesetzt wird.

6. Verfahren nach Anspruch 1 bis 5, dadurch gekennzeichnet, dass man als organische Stickstoff-Base aliphatische Amine einsetzt.

7. Verfahren nach Anspruch 1 bis 5, dadurch gekennzeichnet, dass man als organische Stickstoff-Base aromatische Amine einsetzt.

8. Verfahren nach Anspruch 1 bis 5, dadurch gekennzeichnet, dass man als organische Stickstoff-Base heterozyklische Amine einsetzt.

9. Verfahren nach Anspruch 1 bis 5, dadurch gekennzeichnet, dass man als organische Phosphine aliphatische Phosphine einsetzt.

10. Verfahren nach Anspruch 1 bis 5, dadurch gekennzeichnet, dass man als organische Phosphine aromatische Phosphine einsetzt.

**Revendications**

1. Procédé de préparation de 3,3-dichloro-2-méthyl-propène, caractérisé par le fait que l'on fait réagir du 1-chloro-2-méthyl-propène sur du chlorure de sulfuryle en présence de quantités de bases organiques azotées et/ou de phosphines organiques exerçant un effet catalytique.

2. Procédé selon la revendication 1, caractérisé par le fait que l'on effectue la réaction en phase liquide.

3. Procédé selon les revendications 1 et 2, caractérisé par le fait que l'on effectue la réaction sous la pression normale, à une température de 30 à 68 °C.

4. Procédé selon les revendications 1 à 3, caractérisé par le fait que l'on utilise le chlorure de sulfuryle dans des quantités supérieures ou inférieures à la quantité stoechiométrique, par rapport au 1-chloro-2-méthyl-propène.

5. Procédé selon les revendications 1 à 4, caractérisé par le fait que l'on ajoute la base organique azotée ou la phosphine organique, dans un domaine de concentration de 1 à 10 000 ppm, au 1-chloro-2-méthyl-propène ou que l'on utilise ce dernier avec ces concentrations en bases organiques azotées et/ou en phosphines organiques.

6. Procédé selon les revendications 1 à 5, caractérisé par le fait que l'on utilise, comme base organique azotée, des amines aliphatiques.

7. Procédé selon les revendications 1 à 5, caractérisé par le fait que l'on utilise, comme base organique azotée, des amines aromatiques.

8. Procédé selon les revendications 1 à 5, caractérisé par le fait que l'on utilise, comme base organique azotée, des amines hétérocycliques.

9. Procédé selon les revendications 1 à 5, caractérisé par le fait que l'on utilise, comme phosphines organiques, des phosphines aliphatiques.

10. Procédé selon les revendications 1 à 5, caractérisé par le fait que l'on utilise, comme phosphines organiques, des phosphines aromatiques.

**Claims**

1. A process for the production of 3,3-dichloro-2-methylpropene, characterised in that 1-chloro-2-methyl-propene is reacted with sulphuryl chloride in the presence of a catalytically effective amount of an organic nitrogen base and/or an organic phosphine.

2. A process according to claim 1, characterised in that the reaction is carried out in the liquid phase.

3. A process according to claim 1 or 2, characterised in that the reaction is carried out at 30 to 68 °C under standard pressure.

4. A process according to any of claims 1 to 3, characterised in that the sulphuryl chloride is used in an excess or deficient amount relative to its stoichiometric amount with respect to the 1-chloro-2-methylpropene.

5. A process according to any of claims 1 to 4, characterised in that the organic nitrogen base and/or the organic phosphine is added in an amount of 1 to 10 000 ppm to the 1-chloro-2-methylpropene or that 1-chloro-2-methylpropene containing organic nitrogen base and/or organic phosphine in such a concentration is used.

6. A process accordingto any of claims 1 to 5, characterised in that an aliphatic amine is used as the organic nitrogen base.

7. A process according to any of claims 1 to 5, characterised in that an aromatic amine is used as the organic nitrogen base.

8. A process according to any of claims 1 to 5, characterised in that a heterocyclic amine is used as the organic nitrogen base.

9. A process according to any of claims 1 to 5, characterised in that an aliphatic phosphine is used as the organic phosphine.

10. A process according to any of claims 1 to 5, characterised in that an aromatic phosphine is used as the organic phosphine.